# EUROPEAN PATENT APPLICATION

(11) **EP 2 340 803 A1**
(43) Date of publication of application: **06.07.2011**
(21) Application number: 09180983.0
(22) Date of filing: 30.12.2009
(51) Int. Cl.: A61K 8/02, A61K 8/36, A61K 8/365, A61K 8/368, A61K 8/66, A61K 8/97, A61K 8/98, A61Q 19/00

(54) **Dead skin exfoliating composition and pad for hands and feet**

(71) Applicant: Brands Asia Pacific Limited, Wanchei, Hong Kong (CN)
(72) Inventor: Wang, Chelson Chin-Yuan, Wanchai Hong Kong (CN)
(74) Representative: Lang, Christian

(57) **Abstract**

The present invention provides a dead skin exfoliating composition and pad for hands and feet, comprising a layer of absorption which absorbs a composition of organic acids with dead skin exfoliating effect and pH adjusted at 1.5 to 4.5 with buffer solution of the acid and its salt, and a layer of water-proof barrier to prevent the evaporation of the composition and to increase the absorption of the composition into the dead skin.

## Description

### BACKGROUND OF THE INVENTION

### 1. Technical Field

The present invention relates to a dead skin exfoliating composition and pad for hands and feet which gently and effectively remove cuticles. The pad has a layer of absorption applied with an organic acid composition that serves to remove skin cuticles. The organic acid composition contains components for dead skin exfoliating and cuticles removing, so as to give an excellent antigerm effect, thereby achieving the objective of exfoliating dead skin and removing cuticles optimally.

### 2. Description of Related Art

Dead skin and calluses tend to occur at sole skin due to friction and pressure our feet undergo during our everyday walking. Our hands are also apt to have thick cuticles because of friction in working. Thus, calluse-removing liquid products and cuticles removing foot packs are now commercially available and popular for removing dead skin and calluses on hand and foot skin. The cuticles of our skin are mainly composed of keratin, which dissolves in neither water nor oil, but can be dissolved by strong bases, strong acids and proteases. Therefore, the existing calluse-removing liquid or cuticles removing products are typically made from solution of sodium hydroxide, also known as caustic soda, being a strong base or made from solution of Alpha Hydroxy Acid, a high-level hydroxy acid.

However, either the strong base or the high-level hydroxy acid is somehow irritant and allergy triggering to skin. It is often heard that people have skin allergic, inflamed and even injured skin after using calluse-removing liquids containing strong bases or foot packs made from high-level Alpha Hydroxy Acid. In view of this, there is a need for products that are safe and gentle to our skin and effective in promoting dead skin exfoliation.

Currently, there are various commercially available products for removing dead skin and calluses of hand skin and foot skin. In respect of formulas, there are compositions based on strong bases and Alpha Hydroxy Acid. In respect of administration, some calluse-removing liquid products are to be directly applied to and left on dead skin or calluses for 3 to 5 minutes so that the softened dead skin and cuticles can be then easily removed by rubbing with cuticle removing tools. Some foot cuticle removing packs are made as socks containing therein strong-base calluse-removing liquids or high-level Alpha Hydroxy Acid solution. In use, a user can pack his/her foot with the foot cuticle removing pack for 50 to 120 minutes and rinse the foot skin. After 5 to 7 days, dead skin and cuticles will peel gradually. Due to people's different walking habits, our feet contact the ground with different portions and dead skin as well as calluses can generate at different foot portions with different thickness. Nevertheless, the foregoing foot packs fail to precisely deal with dead skin and calluses that are relatively thick while although the calluse-removing liquid products can be precisely smeared on specific positions, such liquid exposed in the air can easily evaporate and loose its work efficiency. Besides, while the existing calluse-removing liquid products do have effects in dissolving and softening dead skin and cuticles, they fail to make the dissolved or softened dead skin and cuticles come off naturally, but require the assistance of cuticle removing tools or filing boards. In use of such cuticle removing tools or filing boards, in addition to inconvenient operation, there is a risk that our skin may get hurt in case of improper operation. Hence, we do need a novel composition and pad that serve to facilitating exfoliating dead skin and cuticles of hands and feet, wherein the composition and pad are effective and gentle to our skin.

### BRIEF SUMMARY OF THE INVENTION

For a first objective, the present invention comprises three components, namely an organic acid composition for removing dead skin and cuticles, a layer of absorption that is absorptive, and a layer of barrier that is waterproof and airproof. The objective of the present invention is to provide a dead skin exfoliating composition for hands and feet. The composition is effective and gentle and comprises (1) an organic acid and an organic acid salt (2), a degrading enzyme (3), a binding agent (4), a solvent (5), a moisturizing agent and (6) a palliative.

A second objective of the present invention is to provide a dead skin exfoliating composition and pad for hands and feet. The composition and pad are effective and gentle and can evenly cover or pack skin portions to be treated. In the present invention, the foregoing dead skin exfoliating composition for hands and feet is absorbed by an absorptive layer of absorption. The layer of absorption can evenly cover or pack and then evenly and effectively transport the composition to skin portions to be treated.

A third objective of the present invention is to provide a dead skin exfoliating composition and pad for hands and feet, which can evenly cover or pack skin portions to be treated and can disconnect the absorbed composition from the air and external water. The pad is waterproof and airproof, thereby facilitating dead skin and cuticles to absorb the composition.

A fourth objective of the present invention is to provide a skin pad that can pack hands or feet or be attached to specific skin portions, wherein the pad can be cut freely by a user into desired sizes and shapes so as to tightly stick on human bodies and effectively remove skin cuticles of particular human body portions.

### BRIEF DESCRIPTION OF THE SEVERAL VIEWS OF THE DRAWINGS

The invention as well as a preferred mode of use and advantages thereof will be best understood by referring to the following detailed description of the illustrative embodiments in conjunction with the accompanying drawings, wherein:
FIG. 1 is a perspective view of a dead skin exfoliating pad for hands and feet according to the present invention;
FIG. 2 is an exploded view of the dead skin exfoliating pad according to the present invention; and
FIGS. 3, 4 and 5 are schematic drawings showing the dead skin exfoliating pad according to the present invention applied to different portions of a human body.

### DETAILED DESCRIPTION OF THE INVENTION

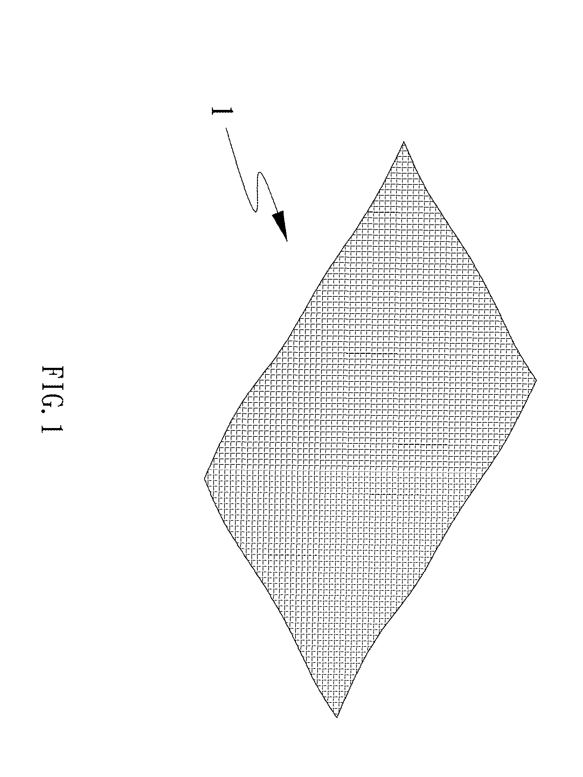

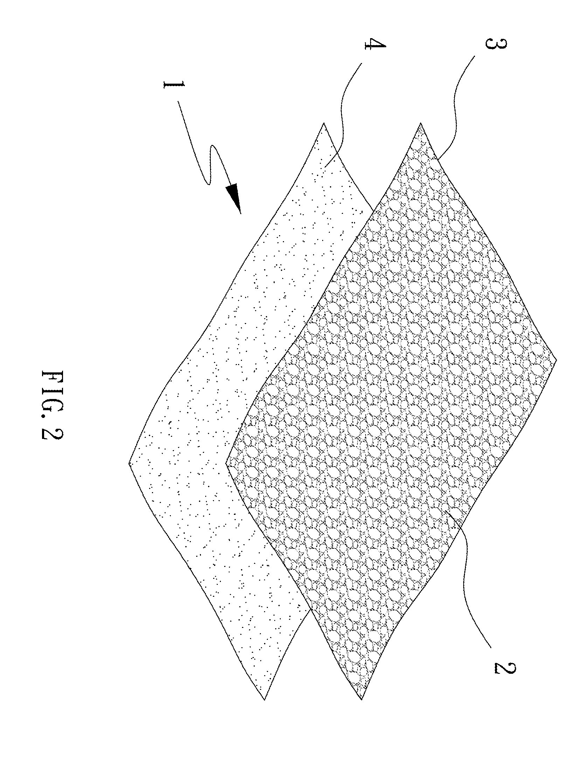

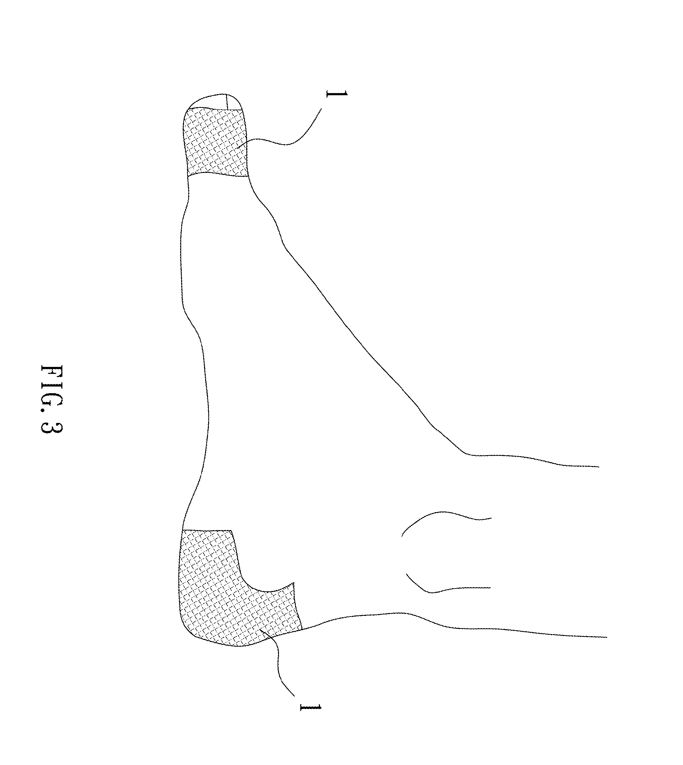

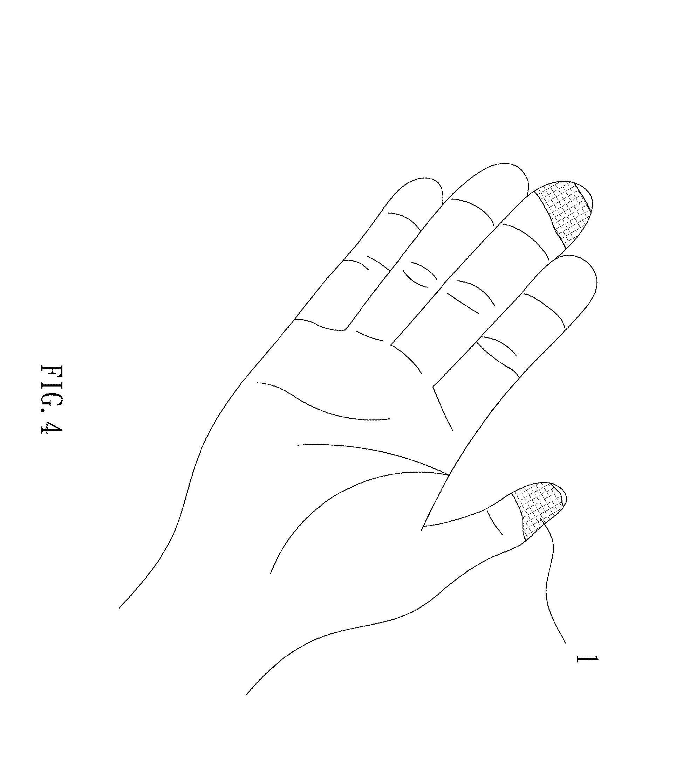

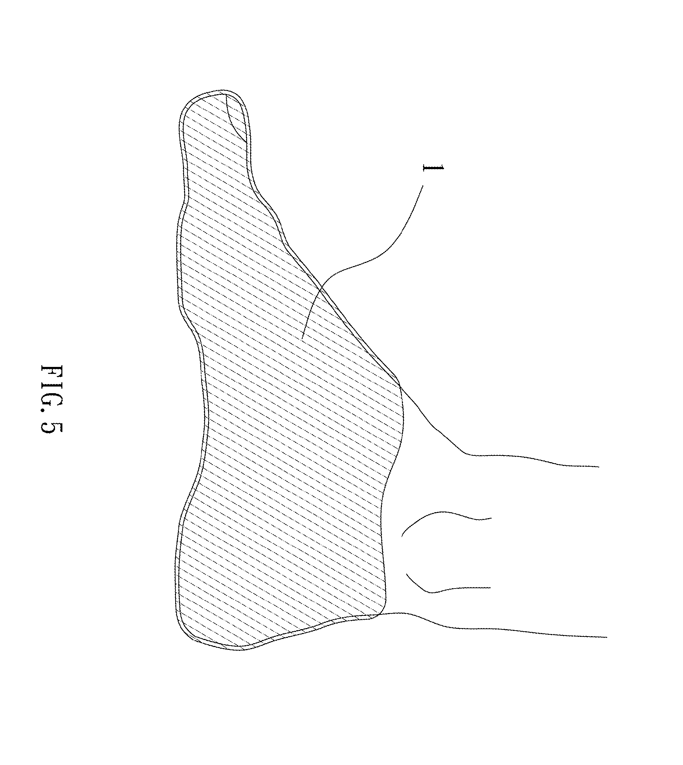

Referring to FIG. 1, FIG. 2, FIG. 3, FIG. 4 and FIG. 5, there are a perspective view, an exploded view and applied drawings of the present invention. Therein, the configuration of a dead skin exfoliating composition and pad 1 for hands and feet is shown. The skin pad 1 has a layer of absorption 3 and a layer of barrier 4, wherein the layer of barrier 4 is waterproof and airproof so as to prevent the organic acid composition from evaporating during use.

The organic acid composition of the present invention is different from the existing commercially available base or acid callus-removing liquid. The present invention adopts a technology named "composite dissolving method for dead skin and cuticles", which is characterized by: (1) having a proper amount of organic acid and its salt to form a buffer solution, (2) having a protease, (3) having a binding agent for thickening and binding, (4) a solvent (5) a moisturizing agent, and (6) a skin palliative. The components will be described in detail below.
I. Organic Acid Composition:
   (1) The buffered solution formed by the organic acid and its salt:
      The organic acid used in the present invention has molecules of a relatively small size and, after dilution, is gentle and harmless to skin. The buffered solution is preferably an aqua solution of a hydroxy acid containing hydroxyl groups. The applicable organic acid containing hydroxyl groups with small molecules may be glycolic acid (also know as hydroxyacetic acid), hydroxypivalic acid (also known as lactic acid), malic acid, citric acid or salicylic acid. The aqua solutions of a salt of any of the above acids are also applicable to form buffered solutions with different pH values. The partially neutralized organic-acid- based buffered solution helps to stabilize the pH value of the solution and is less irritant to skin as compared with unbuffered free acids. However, while the pH value of human skin ranges between 3.5 and 6.0, the pH value of the organic acid must be lower than the pH value of human skin or it is useless in helping dissolving skin cuticles. As long as the pH value of the organic acid is higher than the pH value of human skin, the organic acid has no effect in dissolving skin cuticles. Therefore, in the buffer solution of the present invention, the first component, namely the organic acid and its salt must have a pH value ranging between 1.5 and 4.5 so as to have the function of removing dead skin and cuticles. More preferably, the pH value ranges between 2.5 and 3.5, and the organic acid takes 3% to 30% of the weight of the composition.
   (2) Protease:
      The protease used in the present invention may be one from fruits, such as papain, bromelain and the protease for milk ferment. The protease helps to not only dissolve proteins in dead skin and cuticles, but also reduce irritation of skin caused by the organic acid. The protease in the present invention takes 0.5% to 20% of the weight of the composition.
   (3) Binding Agent:
      The binding agent used in the present invention may be natural xanthan gum or cellulose adhesives, such as hydroxyethyl cellulose or carboxymethyl cellulose. In the composition of the present invention, the binding agent takes 0.5% to 6% of the weight of the composition.
   (4) Solvent:
      The solvent in the present invention may be water, ethanol, or glycerol for dissolving the organic acid and the binding agent, while taking 10% to 60% of the weight of the composition.
   (5) Moisturizing Agent:
      The moisturizing agent in the present invention may be a polyol, such as glycerol, propylene glycol, butyl glycol and diethylene glycol. The moisturizing agent may also be an amino-acid-based moisturizing agent. The moisturizing agent serves to maintain moisture of the buffer solution of the organic acid and
      its salt by preventing water from evaporating so as to maintain the solution at a constant concentration. The moisturizing agent takes 3% to 25% of the weight of the composition.
   (6) Skin Palliative:
      The present invention further comprises the skin palliative, which may be a plant extract, such as aloe extract, marigold extract, chamomile extract, and
      cornflower extract. The skin palliative takes 0.2% to 15% of the weight of the composition.

As to the safety of the organic acid composition for removing skin cuticles according to the present invention, human experimentation was performed in Taiwan on August 10, 2009. Twelve human subjects from Chia Nan University of Pharmacy & Science received tests on human skin irritation, and the results prove that the chemical component brought no irritant reaction body skin and foot skin. None of erythema, urticaria, pruritus, inflammation, pain happened. Table 1 and Table 2 show the results of the tests where the 12 subjects used the samples of skin pads and foot dressing while Table 3 and Table 4 further reflect the tests where the subjects were grouped into three groups, namely 20-to-30 year-old subjects, 30-to-40 year-old subjects, and 40-to-50 year-old subjects for using the samples of skin pads and foot dressing.

**Table 1: Test for Close-Type Skin Pad**

| Number of Subjects | Human Test for Skin Pad | | | | |
|---|---|---|---|---|---|
| | Erythema | Urticaria | Pruritus | Inflammation | Pain |
| 12 | X | X | X | X | X |
| Test Result | None of the above irritant reaction happened | | | | |

**Table 2: Test for Open-Type Foot Dressing**

| Number of Subjects | Human Test for Foot Dressing Directly Applied on Skin | | | | |
|---|---|---|---|---|---|
| | Erythema | Urticaria | Pruritus | Inflammation | Pain |
| 12 | X | X | X | X | X |
| Test Result | None of the above irritant reaction happened | | | | |

**Table 3: Test for Close-Type Skin Pad**

| Human Test for Skin Pad | | Number of Subjects Sheets | Skin Reaction (Erythema, Urticaria, Pruritus, Inflammation, Pain) | | |
|---|---|---|---|---|---|
| | | | Time/Hour | | |
| | | | 24 | 48 | 72 |
| | 20-30 year-old | 4 | None of the above 4 irritant reaction irritant reaction happened | None of the above irritant reaction happened | None of the above irritant reaction happened |
| Age of Subject | 30-40 year-old | 4 | None of the above 4 irritant reaction happened | None of the above irritant reaction happened | None of the above irritant reaction happened |
| | 40-50 year-old | 4 | None of the above irritant reaction happened | None of the above irritant reaction happened | None of the above irritant reaction happened |

**Table 4: Test for Open-Type Foot Dressing**

| Human Test for Foot Dressing Directly Applied on Skin | | Number of Subjects | Skin Reaction (Erythema, Urticaria, Pruritus, Inflammation, Pain) | | |
|---|---|---|---|---|---|
| | | | Time/Hour | | |
| | | | 24 | 48 | 72 |
| | 20-30 year-old | 4 | None of the above 4 irritant reaction happened | None of the above irritant reaction happened | None of the above irritant reaction happened |
| Age of Subject | 30-40 year-old | 4 | None of the above 4 irritant reaction happened | None of the above irritant reaction happened | None of the above irritant reaction happened |
| | 40-50 year-old | 4 | None of the above 4 irritant reaction happened | None of the above irritant reaction happened | None of the above irritant reaction happened |

The table below shows examples of organic acid composition of the present invention containing ingredients of different weight percentages:

| Example | 1 | 2 | 3 |
|---|---|---|---|
| | Weight % | | |
| Ingredient | | | |
| Pure Water | 18.3 | 32.8 | 36.5 |
| Milk Ferment Filtrate | 15 | 18 | 18 |
| Ethanol | 15 | 15 | 5.5 |
| Glycolic Acid (70%) | 15 | -- | 3.5 |
| Lactic Acid (88%) | 8 | 3 | 1.8 |
| Aloe Extract | 5 | 10 | 16 |
| Glycerol | 5 | 9.5 | 5 |
| Sodium Citrate | 5 | 1.5 | 0.7 |
| Bamboo Vinegar | 3 | -- | -- |
| Butyl Glycol | 3 | 3 | 3 |
| Propylene Glycol | 3 | 3 | 3 |
| Salicylic Acid | 1.9 | 1.9 | 1.9 |
| Hydroxyethyl Cellulose | 0.5 | 0.5 | 0.5 |
| Citric Acid | 0.5 | -- | 0.1 |
| Plant Extract | 1.8 | 1.8 | 4.5 |
| Total | 100 | 100 | 100 |
| pH Value of Resultant Composition | 2.8 | 3.8 | 3.6 |

### II. Layer of Absorption 3

Another component of the present invention is the absorptive layer of absorption 3, which is made of nonwoven, dust-free paper or cotton cloth, for absorbing and then releasing the organic acid composition.

### III. Layer of Barrier 4

Another component of the present invention is the waterproof, airproof layer of barrier. The layer of barrier 4 is attached to a side of the layer of absorption 3 that is opposite to a side having the organic acid composition, and the layer of barrier is a thin film of a polymer, such as polyethylene, polypropylene, or polyvinyl chloride.

In use of the dead skin exfoliating composition and pad 1 for hands and feet, after an application for 45 to 60 minutes, the body portion applied with the composition or the pad 1 shall be rinsed with warm water and dried with dry cloth. Skin cuticles will automatically come off in 5 to 10 days. The dressing 2 of the novel composition provides better effects in exfoliating dead skin and removing cuticles removing, while relaxing, moisturizing and enriching skin. An additional feature of the present invention is that the skin pad 1 can be used as hand packs or foot packs, and can also allow a user to freely cut it. In use, a user may choose not to pack his/her whole foot in the pack but to attach a tailored piece of the skin pad 1 at any body portion where there are dead skin and cuticles to be removed. The positions where cuticles happen vary from person to person. For example, a sportsman may usually have his/her heels and soles rubbed due to sudden stop after sprint while a lady may usually have her front soles rubbed because of the high heels. In view of that cuticles may happen at different portions with different thickness, the present invention can directly and precisely treat the portions to be treated by making the skin pads 1 catering for the shapes of heels, soles, and toes, so as to effectively perform dead skin exfoliating and cuticles removing, without hurting skin texture other than the portions to be treated.

The embodiments described above are intended only to demonstrate the technical concept and features of the present invention so as to enable a person skilled in the art to understand and implement the contents disclosed herein. It is understood that the disclosed embodiments are not to limit the scope of the present invention. Therefore, all equivalent changes or modifications based on the concept of the present invention should be encompassed by the appended claims.

## Claims

1. A dead skin exfoliating organic acid composition for hands and feet, the composition comprising (1) an organic acid and its salt jointly taking 3% to 30% of a weight of the composition;(2) a protease taking 0.5% to 20% of the weight of the composition; and (3) a skin palliative taking 0.2% to 10% of the weight of the composition;
wherein the organic acid and its salt form a salt buffered solution in the
composition, and the composition has a pH value ranging between 1.5 and 4.5.

2. The composition of Claim 1, wherein the organic acid is selected from the group consisting of acetic acid, glycolic acid, lactic acid, malic acid, tartaric acid, citric acid, acetic acid, bamboo vinegar, and salicylic acid.

3. The composition of Claim 1, wherein the protease is selected from the group consisting of papain, bromelain, and milk ferment filtrate.

4. The composition of Claim 1, wherein the skin palliative is a plant extract selected from the group consisting of marigold, anthemis nobilis, tilia cordata, cornflower, chamomile, hypericum perforatum, willow bark, and ginkgo leaf.

5. A skin pad comprising the composition of Claim 2, wherein the pad has a layer of absorption and a layer of barrier, the layer of barrier being waterproof and airproof so as to prevent the organic acid composition from evaporating during use.

6. The skin pad of Claim 5, wherein the layer of absorption is made of nonwoven, dust-free paper or cotton cloth for absorbing the organic acid composition and then releasing the organic acid composition therein.

7. The skin pad of Claim 5, wherein the layer of barrier is attached to a side of the layer of absorption that is opposite to a side having the organic acid composition, and the layer of barrier is made of polyethylene, polypropylene, or polyvinyl chloride.
